Europäisches Patentamt

European Patent Office

Office européen des brevets

(19)

(11) Publication number: **0 107 214**
A2

# (12) EUROPEAN PATENT APPLICATION

(21) Application number: 83201263.7

(51) Int. Cl.³: **A 61 K 31/17, A 61 K 31/44**

(22) Date of filing: 02.09.83

(30) Priority: 02.09.82 US 414037
02.09.82 US 414038

(43) Date of publication of application: 02.05.84
Bulletin 84/18

(84) Designated Contracting States: AT BE CH DE FR GB IT
LI NL SE

(71) Applicant: DUPHAR INTERNATIONAL RESEARCH B.V,
C.J. van Houtenlaan 36, NL-1381 CP Weesp (NL)

(72) Inventor: Jenkins, Vernon K., c/o OCTROOIBUREAU
ZOAN B.V. Apollolaan 151, NL-1077 AR Amsterdam (NL)
Inventor: Mayer, Richard T., c/o OCTROOIBUREAU
ZOAN B.V. Apollolaan 151, NL-1077 AR Amsterdam (NL)

(74) Representative: Muis, Maarten, Drs. et al,
OCTROOIBUREAU ZOAN B.V. Apollolaan 151,
NL-1077 AR Amsterdam (NL)

(54) Pharmaceutical compositions containing benzoyl-phenyl ureas with anti-tumour activity.

(57) A method of and composition suitable for combating tumors in mammals is disclosed, characterized in that a therapeutically effective quantity of at least one 1-benzoyl-3-phenyl ureas or a metabolite thereof, which compounds are known per se, is administered in a pharmaceutically acceptable carrier.

EP 0 107 214 A2

ACTORUM AG

Pharmaceutical compositions having antitumor activity.

The present invention relates to novel pharmaceutical compositions having antitumor activity, and to a method of treating tumors in mammals.

United States Patent No. 3.748.356 discloses that 1-benzoyl-3-phenyl ureas have insecticidal activity. This activity is based on a mechanism that is not fully understood at this time. It has been found that the insects are killed by these compounds because these 1-benzoyl-3-phenyl ureas influence, i.e., inhibit, chitin formation.

Furthermore, European Patent Application No. 0.025.363 mentions that N-benzoyl-N'-pyridyloxy phenyl ureas have antitumor effects.

It has now been found that 1-benzoyl-3-phenyl ureas known per se with the exception of the compounds having antitumor activity known from European patent application No. 0.025.363, Japanese patent application No. 185.451 (publication No. 57.109.721) and Japanese patent application No. 104765 (publication No. 57.031.664), which have inhibitory activity on chitin formation and known metabolites thereof, have a very interesting cytostatic or tumoricidal effect on mammalian tumors.

More specifically, it was found that

1) 1-(2,6-difluorobenzoyl)-3-(4-chlorophenyl)urea (known as diflubenzuron);

2) 1-(2,6-difluorobenzoyl)-3-[3,5-dichloro-4-(3-chloro-5--trifluoromethyl-2-pyridyloxy)phenyl]urea;

3) 1-(2,6-difluorobenzoyl)-3-[3-chloro-4-(4-chlorophenoxy)phenyl]urea;

4) 1-(2,6-difluorobenzoyl)-3-(4-trifluoromethylphenyl)urea (known as PH 60-44);

5) 1-(2-chlorobenzoyl)-3-(4-chlorophenyl)urea;

6) 1-(2-chlorobenzoyl)-3-(4-trifluoromethoxyphenyl)urea;

7) 1-(2,6-difluorobenzoyl)-3-(4-chloro-2-hydroxyphenyl urea;

8) 1-(2,6-difluorobenzoyl)-3-(4-chloro-3-hydroxyphenyl)urea

9) 1-(2-chlorobenzoyl)-3-(2-hydroxy-4-trifluoromethoxyphenyl)urea

10) 1-(2,6-difluorobenzoyl)-3-(4-cyanophenyl)urea;

11) 1-(2,6-difluorobenzoyl)-3-[4-(1,1,2,3,3,3-hexafluoropro-
    poxy)phenyl]urea;

12) 1-(2,6-difluorobenzoyl)-3-(4-hydroxyphenyl)urea;

13) 1-(2,6-difluorobenzoyl)-3-(4-cyclohexylphenyl)urea;

14) 1-(2,6-difluorobenzoyl)-3-(4-trifluoromethoxyphenyl)urea

15) 1-(2,6-difluorobenzoyl)-3-(3,4-dichlorophenyl)urea;

16) 1-(2-chlorobenzoyl)-3-(4-trifluoromethylphenyl)urea;

17) 1-(2,6-dichlorobenzoyl)-3-(4-chlorophenyl)urea;

18) 1-(2,6-dichlorobenzoyl)-3-(4-trifluoromethylphenyl)urea

19) 1-benzoyl-3-(4-chlorophenyl)urea;

20) 1-(2,6-difluorobenzoyl)-3-(2-hydroxy-4-trifluoromethylphenyl)urea;

21) 1-(2-chlorobenzoyl)-3-(2-hydroxy-4-trifluoromethylphenyl)urea;

inhibit tumor growth in several test models.

To determine the inhibitory activity of the compounds in vivo, three test models have been used:

I. B-16 malignant melanoma in C57BL/6 mice.
To study the effect of drugs or other agents on growth of
tumors in vivo, a metastatic tumor such as B-16 melanoma
has the advantage of providing both a primary site and
microscopic foci of metastatic tumors.

II. Ridgeway Osteogenic Sarcoma (ROS) in AKR mice.
The advantage of the use of such a localized, non-metastatic tumor for studies on drug effects is that pertuba-
tions of tumor growth and longevity of the mice are attributable to a direct effect on the primary tumor.

III. Skin carcinoma (Ca 1025) in AKR mice.
This tumor represents a second histologic cell type arising from skin and is less malignant than malignant mela-
noma.

0107214

-3-

The compounds were tested by intraperitoneal injections of suspensions of the compounds in an aqueous solution of polyvinyl pyrrolidone. Doses and schedules of injection were varied, but no optimal dose-schedule has as yet been determined for either compound.

The lethality of the compounds wth the exception of PH 60-44 was extremely low. Injection of the compound was essentially non-lethal even in massive doses of 100 mg/mouse.

Lethality for PH 60-44 was observed at a much lower dose. Multiple injections for a total dose of 25-30 mg during a five day period resulted in 10-15% mortality. Therefore, the experiments included injections of 10 mg each for 3 injections or 5 mg each for 5 injections.

The compounds were tested according to the following procedures:

A. C57BL mice with malignant melanomas (B-16)

Mice with malignant melanomas were injected with 20 mg or 40 mg diflubenzuron in suspension or with the suspension medium (blank). In two of the experiments an initial injection of 20 mg of diflubenzuron resulted in an 11.7% and a 13.4% reduction in tumor size within 24 hours of treatment, as compared to a 60.7% and a 34% increase, respectively, in tumor size for blank-treated controls (Table I). This effect is also emphasized by a large increase in doubling time (i.e., mean time in days for the tumors to double in mass calculated on the basis of the change within 48 hours after treatment) from 1.9 days for the blank-treated control to 10.4 days for diflubenzuron--treated animals (Table I). Injection of 40 mg resulted in a greater effect (initial decrease by 22% and a negative doubling time).

-4-

TABLE I*

| treatment | % change in tumor mass | | initial doubling time (in days) |
|---|---|---|---|
| | day 1 | day 2 | 0-2 days |
| 20 mg diflu-benzuron | -11.7(26) | 18.6(26) | 10.4(26) |
| 0.2 ml blank | 60.7(16) | 128.6(16) | 1.9(16) |
| 20 mg diflu-benzuron | -13.4(22) | ----- | |
| 0.2 ml blank | 34.0(9) | ----- | |
| 40 .mg diflu-benzuron | -22.4(8) | -14.7(8) | -6.7(8) |
| 0.4 ml blank | 31.4(9) | 85.1(9) | 2.5(9) |

In a further experiment injections of 20 mg each on day 0 and day 2 or 4 resulted in a further delay of tumor growth (Table II). After a single injection the tumors remained reduced for about 2 days. However, a repeat injection of 20 mg on day 2 or 4 resulted in reduced rate of growth through about the 6th day.

TABLE II

| treatment | % change in tumor mass | | | | | | |
|---|---|---|---|---|---|---|---|
| | day 1 | day 2 | day 3 | day 4 | day 5 | day 6 | day 15 |
| 2 x 20 mg. diflubenzuron | -11.7 (26) | 18.6 (26) | 92.6 (16) | 143 (26) | 178 (10) | 264 (26) | 1187 (16) |
| 2 x 0.2 blank | 60.7 (16) | 128 (16) | 176 (11) | 247 (5) | 314 (16) | 437 (16) | 1402 (14) |

Repeated injections for a total of 100 mg given in 3 or 5 injections within 6 days after the initial in-

---

* For tables I-XV the number of tumors measured is given in parenthesis. Negative values indicate a decrease in tumor size. A negative doubling time shows time for decrease to one-half size.

0107214

-5-

jection reduced tumor growth through the 11th day (Table III). Between days 12 and 19 the tumor growth was apparently but not significantly less than for controls. Treated tumors, however, remained virtually static after the 19th day and the increases for days 20 and 21 were less than for the blank-treated controls.

TABLE III

| treatment | % change in tumor mass | | | | | |
|---|---|---|---|---|---|---|
| | day 1 | day 2 | day 5 | day 7 | day 11 | day 12 |
| 100 mg of diflubenzuron in 3 or 5 inj. | -15.8 (30) | -14.7 (8) | 106 (30) | 279 (18) | 358 (11) | 478 (22) |
| 1.0 ml blank in 3 or 5 inj. | 32.7 (18) | 85.1 (9) | 202 (18) | 444 (9) | 562 (9) | 647 (9) |
| | day 13 | day 15 | day 19 | day 20 | day 21 | |
| | 559 (30) | 864 (30) | 1308 (29) | 1220 (27) | 1460 (27) | |
| | 753 (18) | 1026 (18) | 1589 (16) | 1860 (16) | 2206 (16) | |

Similar experiments as were done with diflubenzuron were carried out with PH 60-44.

The initial injection of PH 60-44 on day 0 into mice with malignant melanomas caused stasis in tumor growth for about 24 hours (Table IV). Second and third injections, on days one and five, respectively, however, had little or no effect on subsequent tumor growth (Table IV). Tumor growth at 5 days or later was unaltered as evidenced by percent increase in tumor mass.

TABLE IV

| treatment | % change in tumor mass | | | | | | |
|---|---|---|---|---|---|---|---|
| | day 1 | day 5 | day 6 | day 7 | day 11 | day 14 | day 21 |
| 3 x 10 mg PH 60-44 | -0.8 (11) | 182 (11) | 240 (11) | 317 (11) | 885 (11) | 1251 (11) | 2834 (11) |
| 3 x 0.1 | 31.8 (10) | 206 (10) | 334 (10) | 403 (10) | 920 (10) | 1292 (10) | 2315 (10) |

-6-

Also similar experiments have been carried out with the above identified compounds 2), 3), 5), 6) and 10) to 19).

Comparisons were made between treated tumors and control tumors with a similar mean mass.

The compounds 3), 5), 6) 10), 11), 12), 15) and 19) were given in doses of 20 mg each on days 0, 1, 2, 3 and 4. Compounds 13), 14), 16), 17) and 18) were given in doses of only 10 mg each on days 0, 1, 2, 3 and 4. Finally compound 2) was given in doses of 20 mg each on days 0, 1 and 2.

The results are summarized in table V.

TABLE V

| Treatment | Mean tumor Mass Day 0 | Mean tumor Mass Day 1 | % Change in Tumor Mass |
|---|---|---|---|
| PVP (17) | 224 | 328 | + 40 |
| comp. 2) (16) | 265 | 191 | - 28 |
| comp. 3) (17) | 137 | 127 | - 4 |
| comp. 5) (14) | 229 | 198 | - 8 |
| comp. 6) (10) | 232 | 229 | - 3 |
| comp.10) (21) | 203 | 183 | - 10 |
| comp.11) (20) | 216 | 193 | - 9 |
| comp.12) (15) | 180 | 148 | - 16 |
| comp.13) (30) | 193 | 205 | + 9 |
| comp.14) (37) | 343 | 359 | + 3 |
| comp.15) (17) | 155 | 151 | - 3 |
| comp.16) (29) | 232 | 231 | 0 |
| comp.17) (28) | 327 | 347 | + 9 |
| comp.18) (32) | 324 | 314 | - 3 |
| comp.19) (19) | 189 | 159 | - 15 |

B. Ridgeway Osteogenic Sarcoma (ROS) in AKR mice.

Doses of diflubenzuron of 20 mg on day 0 (Table VI), 20 mg each on days 0 and 3; and 20 mg each on days 0,

-7-

1, 2, 5 and 6 (Table VII) were used in this test model. As with the malignant melanoma model, a reduced rate of growth was observed 24-48 hours after an initial injection of diflubenzuron (Table VI) but tumor size did not decrease.

TABLE VI

| treatment | % change in tumor mass | |
| | day 1 | day 2 |
| --- | --- | --- |
| 20 mg diflubenzuron | 14.4 (29) | 48.5 (19) |
| 0.2 ml blank | 43.5 (17) | 80.0 (11) |

Repeated injections reduced the rate of tumor growth, but not beyond the approximate times of treatment (Table VII).

TABLE VII

| treatment | % change in tumor mass | | | | | |
| | day 1 | day 2 | day 3 | day 4 | day 5 | day 7 |
| --- | --- | --- | --- | --- | --- | --- |
| 2 x 20 mg diflubenz. | 32.0 (10) | -- | 104 (10) | 213 (10) | 298 (10) | 511 (10) |
| 2 x 0.2 ml blank | 73.7 (6) | -- | 223 (6) | 250 (6) | 354 (6) | 562 (6) |
| 5 x 20 mg diflubenz. | 12.2 (10) | 43.3 (10) | -- | -- | 285 (10) | 423 (10) |
| 5 x 0.2 ml blank | 42.4 (6) | 91.4 (6) | -- | -- | 479 (6) | 697 (6) |

| day 14 | day 21 |
| --- | --- |
| 1682 (10) | 4051 (10) |
| 1847 (6) | 3933 (6) |
| 2495 (10) | 8880 (8) |
| 2349 (6) | 6552 (6) |

The effects were less (or the treatment was not as effective) in this tumor model than in the malignant melanoma model.

A single dose of PH 60-44 resulted in reduced rate of tumor growth at 24 hours (Table VIII). This table shows the results obtained in two experiments in which 30 mg total of PH 60-44 was injected into AKR mice with ROS. However, the schedules of injections were days 0, 1 and 2 for the first one, and days 0, 3 and 5 for the second one. Tumors of the first experiment responded very well resulting in tumor stasis or only a slight increase at 1 and 2 days, and reduced percentage increase through 11 days. On the other hand, the protracted schedule (days 0, 3 and 5) was not effective beyond the initial injection.

The initial effect of treatment with PH 60-44 for both experiments in this model was slightly greater than the initial effect of diflubenzuron in the same model.

TABLE VIII

| treatment | % change in tumor mass | | | | | |
|---|---|---|---|---|---|---|
| | day 1 | day 2 | day 3 | day 6 | day 7 | day 11 |
| 3 x 10 mg PH 60-44 | 10.8 (13) | 2.7 (13) | 17.7 (13) | 196 (13) | 314 (13) | 808 (13) |
| 3 x 0.1 ml | 74.0 (8) | 118 (8) | 155 (8) | 435 (8) | 568 (8) | 1240 (8) |
| 3 x 10 mg PH 60-44 | 0.6 (16) | --- | 58.4 (16) | 217 (16) | 293 (16) | 767 (16) |
| 3 x 0.1 ml blank | 10.7 (6) | --- | 61.1 (6) | 190 (6) | 377 (6) | 789 (6) |

| day 14 | day 21 |
|---|---|
| 1501 (13) | 3524 (13) |
| 1813 (8) | 3385 (8) |
| 1277 (16) | 3452 (16) |
| 1323 (6) | 3586 (6) |

-9-

## C. Skin carcinoma (Ca 1025) in AKR mice.

A single injection of diflubenzuron in this model resulted in a decrease in tumor mass at 24 hours and a decreased rate of growth at 48 hours (Table IX).

TABLE IX

| treatment | % change in tumor mass | |
|---|---|---|
| | day 1 | day 2 |
| 20 mg diflubenzuron | -3.8 (35) | 19.9 (7) |
| 0.2 ml blank | 45.9 (26) | 38.2 (8) |

However, multiple doses did not alter tumor growth significantly beyond the initial injection.

The compound PH 60-44 was also tested in this model. Mice were treated with 5 mg each in suspension or 0.05 ml of medium on days 0, 1, 2, 3 and 4 or with 10 mg in suspension or 0.1 ml of medium on days 0, 1 and 5.

An initial injection of 5 or 10 mg resulted in an 8.2% decrease in tumor mass on day 1 and a second injection on day 1 resulted in a 5.7% decrease by day 2, whereas blank-treated controls increased by 54.4% on day 1 and 76.6% by day 2 (Table X).

Multiple injections during the first 5 days after the initial injection resulted in reduced tumor growth throughout the 21-day testing period.

TABLE X

| treatment | % change in tumor mass | | | | | |
|---|---|---|---|---|---|---|
| | day 1 | day 2 | day 3 | day 4 | day 7 | day 8 |
| 5 x 5 mg or 3 x 10 mg PH 60-44 | -8.2 (17) | -5.7 (11) | 12.0 (11) | 28.7 (11) | 124 (17) | 186 (17) |
| 5 x 0.5 ml or 3 x 0.1 ml blank | 54.4 (16) | 76.6 (3) | 100 (9) | 139 (9) | 465 (16) | 547 (16) |

| treatment | % change in tumor mass | | |
|---|---|---|---|
| | day 14 | day 18 | day 21 |
| | 713 (17) | 1357 (17) | 1889 (17) |
| | 1332 (16) | 2116 (15) | 3001 (15) |

It appears from the results of the above test models that the tumoricidal effect of diflubenzuron is probably dose-schedule dependent. Increased doses and protracted doses of diflubenzuron in the malignant melanoma model enhances and prolongs tumor inhibition.

PH 60-44 has a moderate effect on malignant melanoma tumors in that the tumors fail to grow for 24 hours after a single injection. However, PH 60-44 produced a strong a prolonged effect in Ca 1025 skin tumors.

To test the possibility that one or more of the known metabolites of known insecticidally active 1-benzoyl-3-phenyl ureas have an antitumor activity, a series of experiments were carried out to determine the effects of metabolic function inhibitors and inducers on the action of diflubenzuron in the animal model with malignant melanoma tumors.

Mice of the C57BL/6 strain were injected with 1 x $10^5$ malignant melanoma tumor cells, and tumors of approximately 50 - 500 mg developed in the mice at the sites of injection in about 14 days. Animals with growing tumors were treated with 100 mg of diflubenzuron alone (suspended in an aqueous solution of polyvinylpyrrolidone) or in combination with a metabolic inhibitor or a metabolic inducer according to the schedule given in Table XI.

Cobaltous chloride was used as a metabolic inhibitor. This compound inhibits the monooxygenase activity in liver microsomes.

As metabolic inducers phenobarbital and 3-methylcholanthrene were used.

Phenobarbital is a general mixed function oxidase inducer and increases the metabolism of a wide variety of substances. It causes a large increase in biphenyl 4-hy-

0107214

-11-

droxylase activity, but only a small increase in 2-hydroxylase activity.

3-Methylcholanthrene is an inducer of microsomal mixed function oxidase activity. This is mainly responsible for hydroxylation (oxidation) of aromatic compounds. 3-Methylcholanthrene induces 2-hydroxylase activity but not 4-hydroxylation.

## TABLE XI

### Treatment Schedule and dose

| treatment | day-4 | day-3 | day-2 | day-1 | day 0 | day 1 | day 2 | day 3 |
|---|---|---|---|---|---|---|---|---|
| 100 mg difluben-zuron | -- | -- | -- | -- | 20 mg | 20 mg | 20 mg | 20 mg |
| 4 mg phe-nobarbit. and 100 mg di-flubenzu-ron | 1 mg | 1 mg | 1 mg | 1 mg | 20 mg | 20 mg | 20 mg | 20 mg |
| 6.4 mg 3-mehylcho-lanthrene and 100 mg di-flubenzu-ron | 1.6mg | 1.6mg | 1.6mg | 1.6mg | 20 mg | 20 mg | 20 mg | 20 mg |
| 2.4mg co-baltous chloride and 100 mg di-flubenzu-ron | -- | -- | 1.2mg | 1.2mg | 20 mg | 20 mg | 20 mg | 20 mg |

| day 4 |
|---|
| 20 mg |
| 20 mg |
| 20 mg |
| 20 mg |

-12-

The tumors of the experimental groups were measured and growth rates were compared.

## D. Cobaltous chloride as a metabolic inhibitor.

Treatment with diflubenzuron alone gave a reduction in tumor mass on days 1-3. However, when cobaltous chloride was given before administration of diflubenzuron the inhibitory effect of diflubenzuron was decreased (Table XII).

It can be concluded from this experiment that an action to reduce monooxygenase activity of liver microsomes also reduces the antitumor activity of diflubenzuron. These data support the concept that enzymatic activity in liver chromosomes results in a metabolic product of diflubenzuron that is a more active anti-tumor agent than the parent compound.

### TABLE XII

| Treatment | % change in tumor mass | | | | | | |
|---|---|---|---|---|---|---|---|
| | day 1 | day 2 | day 3 | day 4 | day 5 | day 10 | day 14 |
| 100 mg diflubenzuron | -22.7 (17) | -8.6 (17) | -8.4 (17) | 26.3 (17) | 63.9 (17) | 266 (17) | 378 (12) |
| 100 mg diflubenzuron 2.4 mg cobaltous chloride | 14.4 (16) | 20.9 (12) | 36.4 (12) | 39.3 (12) | 59.7 (12) | 306 (12) | 782 (9) |

## E. Metabolic inducers

### 1. Phenobarbital

If, indeed, effects of diflubenzuron are directly related to enzymatic activity, an enhanced enzymatic activity obtained by administration of an oxidase inducer could increase the antitumor activity of diflubenzuron.

In a first experiment phenobarbital was used to enhance liver metabolism, and consequently to increase the

-13-

enzymatic activity of liver microsomes. Phenobarbital was injected into tumor bearing mice prior to the standard treatment with diflubenzuron.

As shown in Table XIII phenobarbital, which causes a large increase in biphenyl 4-hydroxylase activity, but only a small increase in 2-hydroxylation, did not enhance the antitumor activity of diflubenzuron.

TABLE XIII

| treatment | % change in tumor mass | | | | | | |
|---|---|---|---|---|---|---|---|
| | day 1 | day 2 | day 3 | day 4 | day 5 | day 10 | day 14 |
| 100 mg di-flubenzu-ron | -22.7 (17) | -8.6 (17) | -8.4 (17) | 26.3 (17) | 63.9 (17) | 266 (17) | 378 (12) |
| 4 mg phe-nobarbital 100 mg di-flubenzuron | -19.4 (23) | -10.4 (23) | 0.8 (23) | 21.7 (23) | 72.1 (23) | 293 (23) | 572 (10) |

2. 3-Methylcholanthrene (3-MC)

To determine whether increase of 2-hydroxylation of diflubenzuron enhances the antitumor activity of diflubenzuron, 3-MC was given to tumor-bearing mice before the standard treatment with 100 mg diflubenzuron. 3-MC enhances 2-hydroxylase activity but not 4-hydroxylase activity. As a further control a small group of tumor-bearing mice was given 3-MC alone. 3-MC enhanced the antitumor effects of diflubenzuron throughout the five-day measurement period (Table XIV).

TABLE XIV

| treatment | % change in tumor mass | | | | |
|---|---|---|---|---|---|
| | day 1 | day 2 | day 3 | day 4 | day 5 |
| 100 mg diflu-benzuron | -20.2 (22) | -12.5 (22) | 7.9 (22) | 24.4 (22) | 54.7 (22) |

| treatment | % change in tumor mass | | | | |
|---|---|---|---|---|---|
| | day 1 | day 2 | day 3 | day 4 | day 5 |
| 6.4 mg 3-methylcholanthrene and 100 mg diflubenzuron | -29.2 (34) | -23.5 (34) | -20.6 (32) | -15.1 (29) | 5.7 (21) |
| 6.4 mg 3-methylcholanthrene | 122 (6) | 223 (6) | 364 (6) | 417 (6) | 363 (6) |

It is also clear from the table that 3-MC alone does not inhibit tumor growth.

F. Administration of 2-hydroxy- and 3-hydroxydiflubenzuron

As a further experiment to verify the results of the tests described under A and B, groups of tumor-bearing mice were given 100 mg of diflubenzuron, 100 mg of the 3-hydroxy metabolite, or 100 mg of the 2-hydroxy metabolite, respectively, 20 mg each on day 0, 1, 2, 3 and 4.

The 2-hydroxy metabolite was more effective, for the first 48 hours after treatment, than the parent compound diflubenzuron (Table XV). Moreover, the 3-hydroxy metabolite was not active at all.

TABLE XV

| treatment | % change in tumor mass on | | | | | |
|---|---|---|---|---|---|---|
| | day 1 | day 2 | day 3 | day 4 | day 5 | day 11 |
| 100 mg diflubenzuron | -11.1 (12) | -7.2 (12) | 34.6 (12) | 92 (12) | 192 (12) | 891 (12) |
| 100 mg 3-OH diflubenzuron | 39.5 (12) | 52.4 (12) | 91.2 (12) | 149 (12) | 223 (12) | 930 (12) |
| 100 mg 2-OH diflubenzuron | -23.4 (12) | -17.4 (12) | 25.1 (12) | 74.7 (10) | 127 (10) | 699 (10) |

These results support the previous data which indicate that the 2-hydroxy metabolite of diflubenzuron is more tumoricidal than diflubenzuron itself.

G. Effect of some compounds on malignant melanomas (B-16) in vitro.

-15-

Cells of malignant melanomas B-16 grow as a monolayer. The doubling time of this type of cells in a cell--culture is 12 to 16 hours. For this experiment 6 multi-well tissue culture plates having a flat bottom with a surface area of 6 $cm^2$ appeared to be suitable and have been used.

On day 0 a quantity of melanoma B-16 cells were brought on the cell-culture plates.

The test compounds were sonicated and then incubated without cells for 3 hours at 37°C.

Then the compound to be tested was administered to the plates with melanoma B-16 cells in the desired quantity (each test was done in triplicate). Thereafter the plates were incubated in a $CO_2$-incubator for 20 hours at 37°C. Then the incubation was stopped by removing the culture medium containing the test compound, the cells were washed twice, and fresh culture medium was added.

48 Hours after starting the incubation step the number of cells on each plate was measured by means of a microcell Coulter Counter. The results so obtained have been expressed as "percentage of controll cell growth". The results are summarized in Table XVI.

TABLE XVI

| Comp. No. | Percentage of controll cell growth when tested in an amount of | |
|---|---|---|
| | $5 \times 10^2$ µg/ml | $5 \times 10^3$ µg/ml |
| 1 | 78 | 41 |
| 3 | 33 | 8 |
| 5 | 98 | 86 |
| 8 | 51 | 53 |
| 9 | 100 | 70 |
| 20 | 91 | 81 |
| 21 | 101 | 71 |

In the above experiment the compounds to be tested have been used in the form of a suspension. Since it is not quite clear how much of the active compounds is dissolved, the above results cannot be compared very well.

-16-

Therefore a number of compounds has been dissolved in ethanol. The results so obtained in the same test as described above, i.e. against melanoma B-16 cells are summarized in Table XVII.

TABLE XVII

| Comp. No | Amount of act. comp. (ug/ml) | Percentage of controll cell growth | |
|---|---|---|---|
| | | (solution) | (suspension) |
| 1 | 50 | 48 | 79 |
| 2 | 200 | 72 | 114 |
| 6 | 300 | 95 | 88 |
| 7 | 50 | 39 | 103 |
| 7 | 300 | 0 | 103 |
| 8 | 300 | 0 | 63 |
| 21 | 300 | 43 | 50 |

Ethanol itself has no effect on the growth of melanoma B-16 cells.

WHAT IS CLAIMED IS:

1. A composition having antitumor activity characterized in that it contains at least one known insecticidally active 1-benzoyl-3-phenyl urea or a known metabolite thereof, having antitumor activity, as active component, in association with a pharmaceutically acceptable carrier.

2. A composition according to Claim 1, characterized in that it contains at least one of the compounds

1) 1-(2,6-difluorobenzoyl)-3-(4-chlorophenyl)urea;

2) 1-(2,6-difluorobenzoyl)-3-[3,5-dichloro-4-(3-chloro-5-
-trifluoromethyl-2-pyridyloxy)phenyl]urea;

3) 1-(2,6-difluorobenzoyl)-3-[3-chloro-4-(4-chlorophenoxy)
phenyl]urea;

4) 1-(2,6-difluorobenzoyl)-3-(4-trifluoromethylphenyl)urea;

5) 1-(2-chlorobenzoyl)-3-(4-chlorophenyl)urea;

6) 1-(2-chlorobenzoyl)-3-(4-trifluoromethoxyphenyl)urea;

7) 1-(2,6-difluorobenzoyl)-3-(4-chloro-2-hydroxyphenyl
urea;

8) 1-(2,6-difluorobenzoyl)-3-(4-chloro-3-hydroxyphenyl)urea

9) 1-(2-chlorobenzoyl)-3-(2-hydroxy-4-trifluoromethoxyphenyl)urea.

10) 1-(2,6-difluorobenzoyl)-3-(4-cyanophenyl)urea;

11) 1-(2,6-difluorobenzoyl)-3-[4-(1,1,2,3,3,3-hexafluoropro-
poxy)phenyl]urea;

12) 1-(2,6-difluorobenzoyl)-3-(4-hydroxyphenyl)urea;

13) 1-(2,6-difluorobenzoyl)-3-(4-cyclohexylphenyl)urea;

14) 1-(2,6-difluorobenzoyl)-3-(4-trifluoromethoxyphenyl)urea

15) 1-(2,6-difluorobenzoyl)-3-(3,4-dichlorophenyl)urea;

16) 1-(2-chlorobenzoyl)-3-(4-trifluoromethylphenyl)urea;

17) 1-(2,6-dichlorobenzoyl)-3-(4-chlorophenyl)urea;

18) 1-(2,6-dichlorobenzoyl)-3-(4-trifluoromethylphenyl)urea

19) 1-benzoyl-3-(4-chlorophenyl)urea;

20) 1-(2,6-difluorobenzoyl)-3-(2-hydroxy-4-trifluoromethylphenyl)urea;

21) 1-(2-chlorobenzoyl)-3-(2-hydroxy-4-trifluoromethylphenyl)urea;

3. A method of combating tumors in mammals, characterized in that a therapeutically effective quantity of a known insecticidally active 1-benzoyl-3-phenyl urea or a known metabolite thereof is administered in association with a pharmaceutically acceptable carrier.

4. A method according to Claim 3, characterized in that a therapeutically effective quantity of

1) 1-(2,6-difluorobenzoyl)-3-(4-chlorophenyl)urea;

2) 1-(2,6-difluorobenzoyl)-3-[3,5-dichloro-4-(3-chloro-5--trifluoromethyl-2-pyridyloxy)phenyl]urea;

3) 1-(2,6-difluorobenzoyl)-3-[3-chloro-4-(4-chlorophenoxy)phenyl]urea;

4) 1-(2,6-difluorobenzoyl)-3-(4-trifluoromethylphenyl)urea;

5) 1-(2-chlorobenzoyl)-3-(4-chlorophenyl)urea;

6) 1-(2-chlorobenzoyl)-3-(4-trifluoromethoxyphenyl)urea;

7) 1-(2,6-difluorobenzoyl)-3-(4-chloro-2-hydroxyphenyl urea

8) 1-(2,6-difluorobenzoyl)-3-(4-chloro-3-hydroxyphenyl)urea

9) 1-(2-chlorobenzoyl)-3-(2-hydroxy-4-trifluoromethoxyphenyl)urea

10) 1-(2,6-difluorobenzoyl)-3-(4-cyanophenyl)urea;

11) 1-(2,6-difluorobenzoyl)-3-[4-(1,1,2,3,3,3-hexafluoropropoxy)phenyl]urea;

12) 1-(2,6-difluorobenzoyl)-3-(4-hydroxyphenyl)urea;

13) 1-(2,6-difluorobenzoyl)-3-(4-cyclohexylphenyl)urea;

14) 1-(2,6-difluorobenzoyl)-3-(4-trifluoromethoxyphenyl)urea

15) 1-(2,6-difluorobenzoyl)-3-(3,4-dichlorophenyl)urea;

16) 1-(2-chlorobenzoyl)-3-(4-trifluoromethylphenyl)urea;

17) 1-(2,6-dichlorobenzoyl)-3-(4-chlorophenyl)urea;

18) 1-(2,6-dichlorobenzoyl)-3-(4-trifluoromethylphenyl)urea

19) 1-benzoyl-3-(4-chlorophenyl)urea;

20) 1-(2,6-difluorobenzoyl)-3-(2-hydroxy-4-trifluoromethylphenyl)urea;

21) 1-(2-chlorobenzoyl)-3-(2-hydroxy-4-trifluoromethylphenyl)urea;

is used.

5. 1-Benzoyl-3-phenyl urea compounds having anti-tumor activity.

      6. Compounds according to Claim 5 being

1) 1-(2,6-difluorobenzoyl)-3-(4-chlorophenyl)urea;

2) 1-(2,6-difluorobenzoyl)-3-[3,5-dichloro-4-(3-chloro-5--trifluoromethyl-2-pyridyloxy)phenyl]urea;

3) 1-(2,6-difluorobenzoyl)-3-[3-chloro-4-(4-chlorophenoxy)phenyl]urea;

4) 1-(2,6-difluorobenzoyl)-3-(4-trifluoromethylphenyl)urea;

5) 1-(2-chlorobenzoyl)-3-(4-chlorophenyl)urea;

6) 1-(2-chlorobenzoyl)-3-(4-trifluoromethoxyphenyl)urea;

7) 1-(2,6-difluorobenzoyl)-3-(4-chloro-2-hydroxyphenyl urea

8) 1-(2,6-difluorobenzoyl)-3-(4-chloro-3-hydroxyphenyl)urea

9) 1-(2-chlorobenzoyl)-3-(2-hydroxy-4-trifluoromethoxy phenyl)urea.

10) 1-(2,6-difluorobenzoyl)-3-(4-cyanophenyl)urea;

11) 1-(2,6-difluorobenzoyl)-3-[4-(1,1,2,3,3,3-hexafluoropropoxy)phenyl]urea;

12) 1-(2,6-difluorobenzoyl)-3-(4-hydroxyphenyl)urea;

13) 1-(2,6-difluorobenzoyl)-3-(4-cyclohexylphenyl)urea;

14) 1-(2,6-difluorobenzoyl)-3-(4-trifluoromethoxyphenyl)urea

15) 1-(2,6-difluorobenzoyl)-3-(3,4-dichlorophenyl)urea;

16) 1-(2-chlorobenzoyl)-3-(4-trifluoromethylphenyl)urea;

17) 1-(2,6-dichlorobenzoyl)-3-(4-chlorophenyl)urea;

18) 1-(2,6-dichlorobenzoyl)-3-(4-trifluoromethylphenyl)urea

19) 1-benzoyl-3-(4-chlorophenyl)urea;

20) 1-(2,6-difluorobenzoyl)-3-(2-hydroxy-4-trifluoromethylphenyl)urea;

21) 1-(2-chlorobenzoyl)-3-(2-hydroxy-4-trifluoromethyl-phenyl)urea;